# EUROPÄISCHE PATENTANMELDUNG

(11) **EP 1 591 943 A2**
(43) Veröffentlichungstag der Anmeldung: **02.11.2005**
(21) Anmeldenummer: 05090053.9
(22) Anmeldetag: 04.03.2005
(51) Int. Cl.: G06F 19/00

(54) **Sende- und Empfangsgerät**

(30) Priorität: 30.04.2004 DE 102004023654; 03.09.2004 DE 102004043210
(71) Anmelder: Biotronik GmbH & Co. KG, 12359 Berlin (DE)
(72) Erfinder: Diebold, Michael, 12169 Berlin (DE)
(74) Vertreter: Eisenführ, Speiser & Partner

(57) **Zusammenfassung**

Datenübertragungseinheit zur drahtlosen Kommunikation mit einem elektromedizinischen Implantat und einer Datenerfassungs- und -auswertezentrale (Home Monitoring Service Center) wobei die Datenübertragungseinheit eine in ein Basisgerät, insbesondere in ein Mobiltelefon (200), einführbare elektronische Steckkarte (100) ist, die wenigstens eine drahtlose Datenschnittstelle (140) zum Austausch von Informationen mit einem elektromedizinischen Implantat, eine drahtgebundene Datenschnittstelle (110) zum Basisgerät für den Austausch von Informationen zwischen Steckkarte (100) und Basisgerät, mindestens eine Kontrolleinheit (120) zur Steuerung des Datenaustausches über wenigstens die drahtlose Datenschnittstelle (110) sowie einen durch das Basisgerät über die drahtgebundene Datenschnittstelle (110) lesbaren Schreib/Lesedatenspeicher (130) aufweist, wobei die Kontrolleinheit (120) ausgebildet ist, über die drahtlose Datenschnittstelle (110) von einem externen Gerät empfangene Daten in den Schreib/Lesedatenspeicher (130) zu schreiben.

## Beschreibung

Die Erfindung betrifft eine Datenübertragungseinheit für ein Patientengerät zur drahtlosen Kommunikation mit einem elektromedizinischen Implantat und einer Datenerfassungs- und -auswertezentrale (Home Monitoring Service Center).

Moderne elektromedizinische Implantate, insbesondere Herzschrittmacher, Defibrillatoren und dergleichen, bieten Arzt und Patienten ein Höchstmaß an Sicherheit und Komfort durch sogenannte Home Monitoring Funktionen.

Dabei protokolliert das Implantat Diagnose- und Therapieinformationen oder technische Daten das Implantat selbst betreffend und überträgt diese Informationen über eine drahtlose Datenschnittstelle (Telemetrieschnittstelle) an ein externes tragbares Patientengerät. Von dort werden die Daten über eine zweite Telemetrieschnittstelle an das Home Monitoring Service Center weitergeleitet. Im Home Monitoring Service Center werden die Daten für den Arzt gespeichert und visualisiert. Der Arzt kann sich direkt über den Therapieverlauf und den aktuellen Gesundheitszustand seiner Patienten informieren. Er hat damit die Möglichkeit schnell auf Veränderungen zu reagieren.

Ohne Home Monitoring kann der Arzt diese Informationen nur im Rahmen einer Untersuchung des Patienten abfragen. In kritischen Situationen kann dies zu unerwünschten Verzögerungen im Informationsfluss führen. Zudem ist jede Untersuchung für Arzt und Patienten mit erheblichem Zeitaufwand verbunden. Häufige Untersuchung führen insbesondere für den Patienten zu einer Einschränkung von Mobilität und Lebensqualität.

Mit Home Monitoring werden die Implantatinformationen über das Patientengerät (siehe auch US 6553262, US 5752976) im Hintergrund verschickt, ohne dass der Patient in der normalen Lebensführung eingeschränkt wird; d.h., er hat die Sicherheit des ärztlichen Monitorings ohne die Belastung von häufigen Untersuchungen.

Die mittelbare Datenübertragung vom Implantat zum Home Monitoring Service Center über das tragbare Patientengerät wird vorgenommen, um die benötigte Sendeleistung seitens des Implantates zu senken, dessen Batterien nur zusammen mit dem gesamten Implantat durch einen chirurgischen Eingriff gewechselt werden können. Ein solches System sowie ein Verfahren dieses zu betreiben wird z. B. in US 5,752,976 dargelegt.

Hauptsinn und -zweck des fortlaufenden Home Monitorings stellt die Möglichkeit der schnellen Reaktion auf unerwartete Ereignisse dar, so dass Schaden vom Patienten abgewendet werden kann. Da die Bewegungsfreiheit eines Patienten nicht unnötig eingeschränkt werden soll, wird die Datenübertragung zum Home Monitoring Service Center bevorzugt über ein drahtloses Kommunikationsnetz (GSM, UMTS, CDMA, WLAN, Bluetooth) vorgenommen. Gewöhnlich werden für die mittelbare Datenübertragung Patientengeräte gebaut, bei denen eine Schaltung eines Mobiltelefons und eine Schaltung für die Implantatkommunikation in einem Gerät integriert werden. Dies führt zu einem Patientengerät mit relativ großen Abmessungen, das nur für das medizinisch-technische Home Monitoring des Patienten bzw. seines elektromedizinischen Implantates eingesetzt werden kann.

Problematisch ist nun, dass der Patient immer ein relativ unhandliches Gerät bei sich führen muss. Die Erfahrung bei der Betreuung von Risikopatienten hat gezeigt, dass das Gerät trotz seiner eventuell lebensrettenden Bedeutung häufig vergessen und das fortlaufende Home Monitoring des Patienten somit unmöglich wurde. Führt der Patient das Gerät jedoch tatsächlich immer bei sich, bedeutet es eine Beeinträchtigung seiner Lebensqualität allein schon deshalb, weil er durch seine ständige Präsenz fortlaufend an die Gefährdung durch die Krankheit erinnert wird.

Ebenfalls nachteilig sind die hohen Produktionskosten der in kleinen Stückzahlen hergestellten Patientengeräte, die elektronisch zudem fortlaufend an neue Mobiltelefonschaltungen und -standards angepasst werden müssen. Mit einem herkömmlichen Patientengerät kann der Patient außerdem nur vordefinierte Notfallaktionen auslösen, da es trotz des integrierten Mobiltelefons nicht als solches verwendet werden kann. Möchte der Patient beispielsweise ein Beratungsgespräch mit dem Arzt führen, muss er zusätzlich zum Patientengerät ein Mobiltelefon mit sich führen.

Aufgabe der vorliegenden Erfindung ist deshalb, eine Datenübertragungseinheit einzuführen, die die Kommunikation zwischen einem elektromedizinischen Implantat und einem Home Monitoring Service Center ermöglicht, dabei aber für den Patienten eine geringere Beeinträchtigung seiner Lebensqualität bedeutet und weniger wahrscheinlich vergessen wird, so dass das medizinische Home Monitoring des Patienten weniger häufig unterbrochen ist. Die Datenübertragungseinheit muss die korrekte und sichere Übertragung der Implantatdaten garantieren, kostengünstig zu fertigen sein und ein hohes Maß an Flexibilität bezüglich des Einsatzes in aktuellen und zukünftigen Kommunikationsnetzen bieten.

Erfindungsgemäß wird die Aufgabe durch eine in ein Basisgerät einführbare elektronische Steckkarte gelöst, die wenigstens eine drahtlose Datenschnittstelle zum Austausch von Informationen mit einem elektromedizinischen Implantat, eine drahtgebundene Datenschnittstelle zum Basisgerät für den Austausch von Informationen zwischen Steckkarte und Basisgerät, mindestens eine Kontrolleinheit zur Steuerung des Datenaustausches über wenigstens die drahtlose Datenschnittstelle sowie einen durch das Basisgerät über die drahtgebundene Datenschnittstelle lesbaren Schreib/Lesedatenspeicher umfasst, wobei die Kontrolleinheit ausgebildet ist, über die drahtlose Datenschnittstelle von einem elektromedizinischen Implantat empfangene Daten in den Schreib/Lesedatenspeicher zu schreiben und in dem Schreib/Lesedatenspeicher abgelegte Daten an ein elektromedizinisches Implantat zu senden.

Das Basisgerät wird in den meisten Fällen ein Mobiltelefon sein. Als Mobiltelefone werden im Kontext dieser Anmeldung jedoch sämtliche tragbaren Geräte aufgefasst, die einen Datenaustausch über ein Mobilfunknetz oder ein drahtloses Datennetz erlauben. Ausdrücklich sind auch sog. Smartphones, Personal Digital Assistants (PDAs) etc. unter dem Begriff erfasst, selbst wenn eine Sprachkommunikation gar nicht oder nur als Nebenfunktion des Gerätes vorgesehen ist.

Aus Sicht des Mobiltelefons funktioniert die erfindungsgemäße Datenübertragungseinheit wie eine Speicherkarte, auf der Daten vom oder für das Implantat in bestimmten Dateien abgelegt werden. So ist gewährleistet, dass ein breites Spektrum von Mobiltelefonen die Datenübertragungseinheit problemlos und ohne zusätzliche I/O-Konfiguration oder spezielle Treibersoftware erkennen und betreiben kann. Der Patient kann so ein beliebiges Basisgerät zu einem Patientengerät zum Betrieb mit seinem elektromedizinischen Implantat erweitern. Dazu sind keine besonderen technischen Kenntnisse nötig, weil nur die als elektronische Steckkarte ausgeführte Datenübertragungseinheit in das Mobiltelefon gesteckt werden muss.

Wegen der weitreichenden Verbreitung von Mobiltelefonen kann auf diese Weise ein bereits beim Patienten vorhandenes Mobiltelefon für den Zweck des medizinischen Home Monitorings genutzt werden. So muss der Patient kein zusätzliches Gerät mit sich führen und ist jederzeit durch den betreuenden Arzt kontaktierbar. Zudem kann das als Mobiltelefon mit erfindungsgemäßer Datenübertragungseinheit ausgeführte Patientengerät auch für eine normale Telefonkommunikation genutzt werden, was insbesondere in Notfällen eine schnellere Hilfe durch örtliche Rettungsdienste gewährleistet. Da der Patient in den meisten Fällen bereits daran gewöhnt ist, ein Mobiltelefon mit sich zu führen, wird es weniger wahrscheinlich, dass das Home Monitoring unterbrochen wird, weil der Patient das Gerät versehentlich vergessen hat. Für den Patienten wird die Bequemlichkeit außerdem stark erhöht, da die elektronische Steckkarte kaum zusätzlichen Raum einnimmt und sich nahezu unbemerkt in das Mobiltelefon integriert. Des weiteren reduziert sich der Herstellungs- und Entwicklungsaufwand, da die Funktionalität der Mobilfunkdatenschnittstelle nicht eigens verwirklicht werden muss. Zusätzlich können Kosten gespart werden, da gegebenenfalls nur ein Mobiltelefon betrieben werden muss. Das Home Monitoring von Patienten kann einfach über alternative oder neue drahtlose Kommunikationsnetze durchgeführt werden, indem das die elektronische Steckkarte aufnehmende Mobiltelefon durch ein entsprechendes Gerät ausgetauscht wird.

Die Aktualisierung der Software für den Betrieb der Datenübertragungseinheit in einem Mobiltelefon oder eine Änderung der Betriebsparameter des elektromedizinischen Implantats kann einfach im Beisein des betreuenden Arztes über eine vom Mobiltelefon ausgehende Datenverbindung durchgeführt werden. Bisher waren dafür spezielle Programmiergeräte notwendig, über die das Implantat mittels Nahfeldtelemetrie konfiguriert wurde. Dabei erfordert jedoch jedes Fabrikat ein auf dieses abgestimmtes Programmiergerät des Herstellers des Implantats, da keine standardisierte Schnittstelle für die Programmierung von elektromedizinischen Implantaten existiert. Da ein Arzt jedoch schon aus Platzgründen nur mit einer begrenzten Anzahl von Programmiergeräten, die zudem jeweils Eigenheiten bei der Bedienung aufweisen, konfrontiert werden möchte, müsste er sich zum Nachteil seiner Patienten auf den Einsatz von Fabrikaten einzelner Hersteller von elektromedizinischen Implantaten beschränken.

Es ist wichtig, die Daten, die zwischen Implantat und Home Monitoring Service Center übertragen werden, zu schützen. Dabei muss mehreren Gesichtspunkten Rechnung getragen werden. Neben allgemeinen Aspekten wie dem Datenschutz der patientenbezogenen medizinischen Daten, gelten insbesondere Sicherheitsaspekte. Da dem Home Monitoring Service Center unter anderem die Aufgabe zufällt, aufgrund der vom elektromedizinischen Implantat empfangenen Daten ggf. Alarm auszulösen, ist es für eine korrekte Analyse des medizinischen Zustandes des Patienten unerlässlich, dass die Daten korrekt übermittelt werden. Noch wichtiger ist aber die Übermittlung von Daten an das Patientengerät und das Implantat. Werden für einen bestimmten Patienten geeignete Betriebsparameter beispielsweise für einen Herzschrittmacher wie minimale und maximale Herzschlagfrequenz oder die geeignete Stimulationsspannung vom Home Monitoring Service Center aus den zuvor übermittelten medizinischen Daten ermittelt oder soll eine aktualisierte Version der Betriebssoftware der Datenübertragungseinheit in einem Mobiltelefon an dieses übermittelt werden, muss die korrekte Übertragung dieser neuen Betriebsparameter an das elektromedizinische Implantat bzw. der Betriebssoftware an das Mobiltelefon sichergestellt sein. Kämen verfälschte Betriebsparameter im Implantat zum Einsatz, wäre das Leben des betroffenen Patienten gefährdet. Eine fehlerhaft übertragene Betriebssoftware könnte Fehlfunktionen beim Betrieb von Mobiltelefon und Datenübertragungseinheit bis hin zur Unbenutzbarkeit des Mobiltelefons nach sich ziehen. Deshalb sollen an jedem Punkt der Übertragungskette die aus der Nachrichtentechnik bekannten Kodiermethoden eingesetzt werden, um Übertragungsfehler detektieren oder korrigieren und eine maximale Sicherheit bei der Datenübertragung erreichen zu können. Um zusätzlich sicherzustellen, dass die Betriebsparameter oder -Software von einer autorisierten Quelle stammen, nämlich vom Home Monitoring Service Center, sollen die übermittelten Daten auch noch durch wirksame kryptographische Verschlüsselung geschützt werden. Beispielsweise durch einen oder mehrere in der Datenübertragungseinheit abgelegte und vor Austausch geschützte geheime Schlüssel kann auf diese Weise eine versehentliche oder vorsätzliche Umprogrammierung des elektromedizinischen lmplantates bzw. des Mobiltelefons wirksam verhindert werden. Allerdings kann der Aufwand für eine eigene Dekodier- und Entschlüsselungseinheit im elektromedizinischen Implantat zu hoch sein, so dass die Daten auf der Kurzstreckenübertragung zwischen der Datenübertragungseinheit und dem elektromedizinischen Implantat unverschlüsselt übertragen werden müssten. Dies kann insbesondere durch die unabdingbare niedrige Stromaufnahme des Implantats unvermeidbar werden. In einem solchen Fall ist es unter Umständen aus Sicherheitsgründen oder aufgrund geltender Sicherheitsvorgaben vorzuziehen bzw. unerlässlich, nur das Auslesen von Daten vom Implantat vorzusehen, so dass dieses auf keinen Fall von außen durch Vorgabe geänderter Betriebsparameter beeinflusst werden kann.

Bevorzugterweise wird der Schreib/Lesedatenspeicher der elektronischen Steckkarte als nichtflüchtiger Schreib/Lesedatenspeicher ausgeführt, weil so die zuletzt übertragenen und in der Steckkarte gespeicherten Daten auch nach Abschalten der Betriebsspannung erhalten bleiben. Dadurch kann der Verlust von Daten verhindert werden, wenn beispielsweise der Akku des Mobiltelefons entladen ist.

Weil die elektronische Steckkarte gemäß der Aufgabe in ein marktübliches Mobiltelefon einführbar sein soll, folgt sie vorzugsweise wenigstens hinsichtlich ihrer Abmessungen und der drahtgebundenen Datenschnittstelle zum Mobiltelefon den maßgeblichen Standards, insbesondere den MMC-, SD-, SM-, MemoryStick-und CF-Standards.

Eine Variante der Datenübertragungseinheit verfügt über eine Kodiereinheit, die medizinische oder technische Betriebsdaten des elektromedizinischen Implantats vor deren Übertragung an das Home Monitoring Service Center verschlüsselt, um die Sicherheit der übertragenen Daten vor unbefugtem Zugriff zu gewährleisten und den Austausch der übertragenen Daten durch verfälschte Daten zu verhindern. Dem Datenschutzerfordernis einiger Länder wird auf diese Weise ebenfalls Rechnung getragen. Die Kodiereinheit kann durch geeignete Programmierung der als Mikrokontroller verwirklichten Kontrolleinheit der Karte realisiert sein.

Die Datenübertragungseinheit kann auch eine Dekodiereinheit besitzen, die Daten, die vom Home Monitoring Service Center verschlüsselt übertragen wurden, entschlüsselt. Diese Ausführung hat den Vorteil, dass auch die Datenübertragung vom Home Monitoring Service Center zur Datenübertragungseinheit vor unbefugtem Zugriff geschützt ist. Auch die Dekodiereinheit kann durch geeignete Programmierung der als Mikrokontroller verwirklichten Kontrolleinheit der Karte realisiert sein.

In einer Variante der letzten Ausführung ist die Dekodiereinheit außerdem dazu ausgebildet, vom Home Monitoring Service Center empfangene Betriebsparameter für das elektromedizinische Implantat auf ihre Richtigkeit zu überprüfen, bevor sie an das Implantat übertragen werden. Dadurch kann eine fehlerhafte Programmierung des Implantats, die eine Gefährdung der Gesundheit des Patienten bedeuten würde, verhindert werden.

In ähnlicher Art und Weise kann eine Fehlprogrammierung des Mobiltelefons, in dessen Steckkartenplatz die Datenübertragungseinheit betrieben wird, verhindert werden, indem bei einer alternativen Ausführung der Erfindung die Dekodiereinheit derart ausgelegt wird, dass sie die korrekte Übertragung von Software für den Betrieb der Datenübertragungseinheit in dem Mobiltelefon vom Home Monitoring Service Center zum Patientengerät durch ein Prüfen des Ergebnisses bei der Dekodierung oder Entschlüsselung der empfangenen Betriebssoftware sicherstellt.

Die drahtlose Schnittstelle kann mit einem oder mehreren elektromedizinischen Implantaten und anderen Sensoren kommunizieren. Die Frequenzbänder, die für die drahtlose Übertragung von Daten zur Verfügung stehen, sind durch internationale Vereinbarungen und nationale Bestimmungen vorgeschrieben. Die drahtlose Datenschnittstelle der elektronischen Steckkarte ist deshalb vorteilhafterweise ausgebildet, eine Datenübertragung bei mindestens einer solchen freigegebenen Frequenz gemäß den geltenden Bestimmungen durchführen zu können. Besonders bevorzugt soll sie Datenübertragungen im Frequenzbereich von 401 bis 406 MHz durchführen können.

Es kann in einer Ausführung vorteilhaft vorgesehen sein, ein Beschreiben des Schreib/Lesedatenspeichers über die drahtgebundene Datenschnittstelle unmöglich zu machen, so dass die elektronische Steckkarte statt wie normalerweise zur bidirektionalen Kommunikation zwischen Implantat und Home Monitoring Service Center nur für die vom Implantat ausgehende unidirektionale Kommunikation eingesetzt werden kann. Auf diese Weise kann verhindert werden, dass die vom elektromedizinischen Implantat gesandten Daten vor der Weitergabe an das Home Monitoring Service Center etwa durch fehlerhafte Software des Mobiltelefons oder unsachgemäßen oder unautorisierten Fremdeingriff verändert oder überschrieben werden und dadurch eventuell aufgrund der veränderten Daten ein Fehlalarm ausgelöst oder gar ein echter Alarm unterbunden wird.

In einer alternativen Ausführung wird das Beschreiben des Schreib/Lesedatenspeichers über die drahtgebundene Datenschnittstelle hingegen ermöglicht, um auch eine Übermittlung von Informationen (Konfigurationsparametern etc.) vom Home Monitoring Service Center an das elektromedizinische Implantat vornehmen zu können.

Der Schreib/Lesedatenspeicher kann in einer bevorzugten Ausführung als sog. dual-ported RAM, also als gleichzeitig über zwei verschiedene Schnittstellen ansprechbarer Speicher mit wahlfreiem Zugriff, aufgebaut sein. Vorzugsweise ist der Schreib/Lesedatenspeicher linear adressierbar.

In einer besonders bevorzugten Ausführung ist der Schreib/Lesedatenspeicher zwischen die drahtlose und die drahtgebundene Datenschnittstelle geschaltet. Eine direkte Weitergabe der Daten von der drahtlosen zur drahtgebundenen Datenschnittstelle und umgekehrt ist in dieser Ausführung nicht möglich.

Da die korrekte und vollständige Übermittlung der Daten zum und ggf. vom Home Monitoring Service Center wichtig ist, wird in einer Ausführung der erfindungsgemäßen Idee ein geeigneter Energiespeicher oder -puffer vorgesehen, der die Stromversorgung der elektronischen Steckkarte wenigstens kurzzeitig aufrecht erhalten kann, so dass bei einer plötzlichen Unterbrechung der Stromversorgung durch das Mobiltelefon nach Möglichkeit eine laufende Übertragung von Daten noch beendet werden kann.

Um zu verhindern, dass die elektronische Steckkarte versehentlich aus dem Mobiltelefon entfernt wird, während eine Datenübertragung durchgeführt wird, weist eine bevorzugte Ausführung der Datenübertragungseinheit eine optische Anzeige auf, der die Durchführung einer Datenübertragung signalisiert (Link-Indikator).

Eine solche Anzeige ist auch in manchen Ländern aus datenschutzrechtlichen Gründen oder durch Bestimmungen zur Funkdatenübertragung vorgeschrieben und ist auch bei eingesteckter elektronischer Steckkarte sichtbar. Alternativ kann die optische Anzeige auch über das Display des Mobiltelefons durch geeignete Programmierung der Betriebssoftware vorgenommen werden.

Soll die elektronische Steckkarte auch von Sehbehinderten benutzt werden können, kann eine alternative Ausführung auch über einen akustischen Signalgeber verfügen, um die Durchführung einer Datenübertragung akustisch zu signalisieren. Alternativ kann auch ein akustischer Signalgeber des Mobiltelefons durch geeignete Programmierung der Betriebssoftware genutzt werden.

In einer besonders bevorzugten Ausführung der Erfindung ist die elektronische Steckkarte derart aufgebaut, dass sie sich seitens des Mobiltelefons wie eine gewöhnliche Datenspeicherkarte ansprechen und steuern lässt. In diesem Fall ist vorteilhafterweise keine angepasste oder eigens programmierte Treibersoftware zur Kontrolle der Steckkarte notwendig. Die Software des Mobiltelefons benutzt in einer solchen Konfiguration die Treibersoftware für eine gewöhnliche Datenspeicherkarte, um Dateien auf der Datenspeicherkarte abzulegen oder auf Dateien lesen oder schreibend zuzugreifen. Die Treibersoftware greift dann direkt schreibend oder lesend auf bestimmte Speicherbereiche einer Datenspeicherkarte zu und veranlasst die gewünschte Dateioperation durch entsprechende Steuerung der drahtgebundenen Datenschnittstelle. Da sich die erfindungsgemäße elektronische Steckkarte als normale Datenspeicherkarte zu erkennen gibt, können seitens des Mobiltelefons wenigstens bestimmte Aktionen der elektronischen Steckkarte durch einen schreibenden oder lesenden Zugriff auf Dateien auf der vermeintlichen Datenspeicherkarte ausgelöst und gesteuert werden, ohne dass eine Anpassung des Betriebssystems des Mobiltelefons etwa durch eine spezielle Treibersoftware vorgenommen werden müsste. Dabei ist es irrelevant, ob auf der elektronischen Steckkarte tatsächlich Speicher in der der gewöhnlichen Treibersoftware für Datenspeicherkarten angezeigten Größe und Beschaffenheit vorliegt.

Fallen Auslesen und Beschreiben des Schreib/Lesedatenspeichers durch die beiden Datenschnittstellen zeitlich zusammen, kann es zu einer Verfälschung der ausgelesenen Daten kommen, da sich diese dann unter Umständen aus Daten zusammensetzen, die teilweise schon vor dem aktuellen Schreibzugriff vorlagen, andernteils durch den in Durchführung befindlichen Schreibzugriff bereits verändert wurden. Eine bevorzugte Ausführung der Erfindung sieht deshalb eine Kontrolleinheit vor, die ausgebildet ist, ein gleichzeitiges oder zeitlich überlappendes Auslesen und Beschreiben zu verhindern oder dergestalt zu koordinieren, dass die ausgelesenen Daten entweder denen entsprechen, die vor dem Schreibzugriff im Datenspeicher vorlagen, oder aber jenen, die gerade in den Datenspeicher geschrieben werden. Alternativ könnten aufeinanderfolgende Schreibzugriffe an unterschiedlichen, nicht überlappenden Adressen des Schreib/Lesedatenspeichers vorgenommen werden, so dass sich die Daten zweier aufeinanderfolgender Schreibzugriffe niemals gegenseitig überschreiben können.

Eine alternative, bevorzugte Ausführung verfügt über einen zweiten Schreib/Lesedatenspeicher, der eine Kopie der in einem Teil des ersten Schreib/Lesedatenspeichers enthaltenen Daten, der durch einen in Durchführung befindlichen Schreibzugriff überschrieben werden soll, für einen gleichzeitigen Lesezugriff bereitstellt.

Fühlt sich ein Patient unwohl, soll er in der Lage sein, eine sofortige Übermittlung von aktuellen medizinischen Daten an das Home Monitoring Service Center auszulösen. Zu diesem Zweck sieht eine bevorzugte Ausführung der Erfindung ein Bedienelement wie z.B. einen Taster vor, durch das eine Übermittlung von Daten ausgelöst werden kann.

In einer bevorzugten Ausführung der Erfindung verfügt die elektronische Steckkarte über ein ROM (Nurlesespeicher) oder Flash-ROM, das Programminstruktionen und -daten zur Ausführung durch eine Kontrolleinheit der elektronischen Steckkarte enthält. In einer bevorzugten Variante enthält das ROM oder Flash-ROM außerdem oder stattdessen Programminstruktionen und -daten zur Ausführung durch das Mobiltelefon. Dadurch steht ein Programm z.B. zur Steuerung der Kommunikation mit dem Implantat nach dem Anschalten der Betriebsspannung sofort zur Verfügung und kann in das Mobiltelefon geladen werden oder in der elektronischen Steckkarte selbst zur Ausführung durch eine Kontrolleinheit gelangen.

Eine weitere Variante der erfindungsgemäßen elektronischen Steckkarte verfügt über ein RAM, das bei der Ausführung von Programminstruktionen anfallende Daten aufnimmt. Die Daten können zum einen bei der Ausführung von Programminstruktionen durch die Kontrolleinheit, oder zum anderen durch das Mobiltelefon anfallen.

Die Erfindung wird im folgenden anhand eines Ausführungsbeispiels mit Bezug auf die Abbildungen näher beschrieben.
Abb. 1 gibt einen Überblick über die elektronischen Komponenten einer erfindungsgemäßen elektronischen Steckkarte und des Mobiltelefons als Basisgerät für den Betrieb der Steckkarte
Abb. 2 zeigt ein Blockdiagramm der Softwarekomponenten von Steckkarte und Mobiltelefon
Abb. 3 zeigt eine Realisierung der Datenübertragungseinheit in der Aufsicht. Die Abmessungen erfüllen die jeweils gültigen Normen der Multifunktionskarten (SD, MMC, usw.)
Abb. 4 zeigt die elektronische Steckkarte in der Seitenansicht

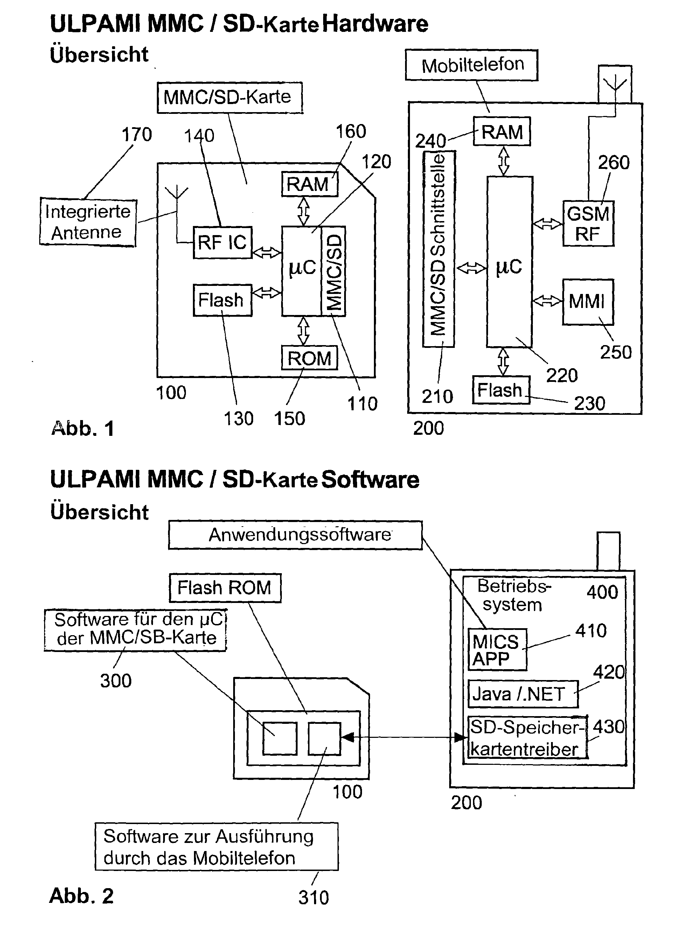

Abb. 1 zeigt Blockdiagramme einer elektronischen Steckkarte 100 gemäß den MMC- und SD-Standards (links) und eines Mobiltelefons 200 (rechts). Die Abmessungen von MMC- und SD-Karten sind ähnlich oder identisch, so dass viele Mobiltelefone über den gleichen Einschub MMC- und SD-Karten aufnehmen können. Die MMC/SD-Karte 100 umfasst eine Antenne 170, einen RAM 160, einen ROM 150, einen drahtlosen Datenschnittstellenbaustein 140, einen als nichtflüchtiger Flash-Speicher ausgeführten Schreib/Lesedatenspeicher 130, eine Kontrolleinheit 120 und eine drahtgebundene Datenschnittstelle 110, die in der Abbildung1 durch sieben entsprechende Blöcke dargestellt sind. Der drahtlose Datenschnittstellenbaustein 140 enthält die zur Kommunikation mit elektromedizinischen Implantaten benötigten Komponenten. Der mit "µC" bezeichnete Block beinhaltet eine als Mikrocontroller ausgeführte programmierbare Kontrolleinheit 120, die verschiedene Steueraufgaben auf der MMC/SD-Karte ausübt. So steuert sie insbesondere auch den Datenaustausch über die als "MMC/SD" bezeichnete drahtgebundene Schnittstelle 110 zum Mobiltelefon 200 oder übernimmt das Ver-und Entschlüsseln der Implantatdaten.

In der im Blockdiagramm dargestellten Ausführung der MMC/SD-Karte ist der Mikrocontroller 120 zentral angeordnet und kommuniziert mit den anderen Blöcken über bidirektionale Busse, die als Pfeile dargestellt sind. Da Mikrocontroller im wesentlichen programmierbare Kontrolleinheiten darstellen, erlaubt dieser Aufbau, mit nur einer zentralen Komponente sowohl Beschreiben und Auslesen des Schreib/Lesedatenspeichers 130, als auch die Kommunikation über die drahtlose Datenschnittstelle 140 zu kontrollieren und durchzuführen. ROM 150 und RAM 160 stehen dabei dem Mikrocontroller 120 zur Seite und enthalten den Programmcode für den Mikrocontroller 120 bzw. nehmen bei dessen Abarbeitung entstehende Daten auf. Anstelle eines festverdrahteten ROMs kann auch ein anderer nichtflüchtiger Speicher vorgesehen sein, so z.B. ein Flash-ROM. Gängige Mikrocontroller beinhalten oft RAM und einen nichtflüchtigen Speicher zur Aufnahme von Programmcode, so dass der Systemaufbau vereinfacht wird. Wird ein solcher Mikrocontroller auf der elektronischen Steckkarte 100 benutzt, sind die dargestellten ROM- und RAM-Blöcke 150 und 160 nicht gesondert verwirklicht.

Das zweite Blockdiagramm zeigt einige zentrale Komponenten eines Mobiltelefons 200. Es dient lediglich zur Veranschaulichung eines typischen Gerätes, das eine elektronische Steckkarte 100 aufnehmen soll.

Auch das Mobiltelefon 200 verfügt über eine als Mikrocontroller (µC) ausgeführte Kontrolleinheit 220, Flash-Memory 230, RAM 240 sowie eine drahtlose GSM-Datenschnittstelle 260 samt Antenne. Des weiteren ist eine drahtgebundene Datenschnittstelle 210 zur MMC/SD-Karte links durch den Block "MMC/SD Interface" dargestellt. Eine Funktionseinheit, die so oder ähnlich nicht auch in der MMC/SD-Karte vorhanden ist, ist ein "Man-Machine-Interface" 250 (MMI), also eine Benutzerschnittstelle mit Anzeige und Tastenfeld. Die als "GSM RF" bezeichnete drahtlose GSM-Datenschnittstelle 260 ermöglicht die drahtlose Kommunikation zwischen Mobiltelefon 200 und einer GSM-Basisstation.

Abb. 2 zeigt ein ähnliches Diagramm wie die Abb. 1. In ihr sind wiederum die MMC/SD-Karte 100 und ein Mobiltelefon 200 schematisch dargestellt. Die Blöcke repräsentieren diesmal jedoch die Software-Komponenten, die zum Betrieb benötigt werden. Auf der elektronischen Steckkarte 100 beinhaltet das Flash-ROM 130 zum einen Software für den Mikrocontroller 120 der Steckkarte 100 (300), zum anderen die Software, die im Mobiltelefon zur Ausführung gelangen soll (310). Diese Software 310 wird von der Steckkarte 100 ins Mobiltelefon 200 geladen und dort vom µC 220 ausgeführt.

Die Softwarekomponenten des Mobiltelefons 200 sind hierarchisch geordnet von oben nach unten dargestellt. Vor dem Hintergrund eines Betriebssystems 400 des Mobiltelefons 200, hier als "Phone OS" bezeichnet, das die gesamte zur Ausführung kommende Software sowie die Hardware kontrolliert, ist dies zu oberst die Anwendungssoftware 410 für den Betrieb der MMC/SD-Karte und eine eventuelle Bedienoberfläche für den Patienten ("MICS APP"). Darunter ist ein JAVA- bzw. ".NET"-Interpreter 420 dargestellt, der den Programmcode der in einer der beiden Sprachen geschriebenen Anwendungssoftware durch Interpretation zur Laufzeit zur Ausführung bringt ("Java/.NET"). Welche Sprache zum Einsatz kommt, hängt dabei vom verwendeten Mobiltelefontypen ab. Alternativ könnten auch dedizierte Programme für ein bestimmtes Mobiltelefon eingesetzt werden, die nicht von einem Interpreter, sondern direkt durch das Mobiltelefon ausgeführt würden.

Logisch zu unterst ist die Treiber-Software 430 für eine SD-Speicherkarte angesiedelt und entsprechend unter den anderen Blöcken eingetragen, vermittels derer das Betriebssystem 400 des Mobiltelefons 200 mit der elektronischen Steckkarte 100 kommuniziert ("SD Memory Card Driver"). Diese Treiber-Software 430 ist standardmäßig in allen Mobiltelefonen enthalten, die Aufnahme und Betrieb einer MMC/SD-Speicherkarte erlauben. Da sich die elektronische Steckkarte 100 auf Seite des Mobiltelefons 200 wie eine normale Speicherkarte verhält, kann zum Betreiben der Steckkarte 100 dieselbe Treiber-Software 430 wie für herkömmliche Speicherkarten benutzt werden. Dadurch muss nicht eigens eine neue Treiber-Software für jeden möglicherweise zum Einsatz kommenden Mobiltelefontypen programmiert werden, was den großen Vorteil einer Ausführung der Erfindung gemäß Anspruch 16 darstellt.

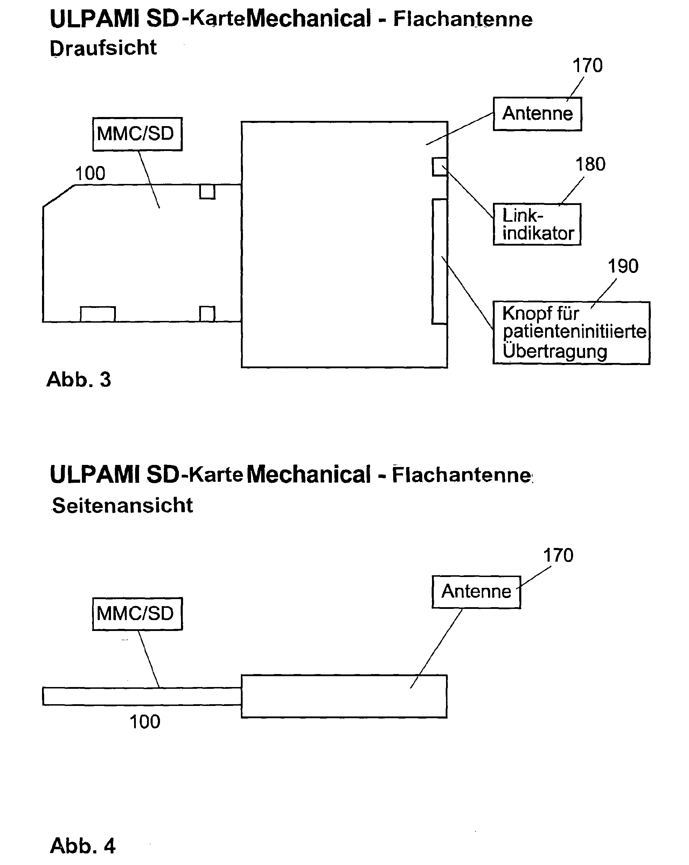

Abb. 3 zeigt eine Ansicht einer elektronischen Steckkarte 100 gemäß dem SD-Standard. Die Steckkarte 100 teilt sich in einen in das Mobiltelefon 200 einführbaren, linkks daregstellten Teil und einen etwas größeren, aus dem Gerät herausragenden Teil auf der rechten Seite der Darstellung. Der links dargestellte Teil folgt den Vorgaben zu den Abmessungen einer SD-Karte. Der rechts dargestellte Teil weist eine rote Leuchtdiode auf, die oben rechts dargestellt ist und einen Link-Indikator 180 bildet, der gemäß Unteranspruch 14 die verschiedenen Betriebszustände der SD-Karte, insbesondere aber eine in Durchführung befindliche Datenkommunikation zwischen dem elektromedizinischen Implantat und der SD-Karte 100 signalisiert. Des weiteren ist am rechts dargestellten Ende der Steckkarte 100 ein Taster 190 vorgesehen, dessen Betätigung gemäß Unteranspruch 19 eine Übertragung von Daten an das Home Monitoring Service Center auslöst. Nicht zu sehen sind Kontakte der drahtgebundenen Datenschnittstelle 110, die am linken Ende auf der Unterseite der Steckkarte 100 angebracht sind. Die gezeigte Variante verfügt über eine Flachantenne 170, die in den aus dem Mobiltelefon herausragenden Teil integriert ist.

Abb. 4 bietet eine Seitenansicht der als SD-Steckkarte ausgeführten Datenübertragungseinheit 100. Auf der links dargestellten Seite befindet sich wiederum der dünne, in das Mobiltelefon einführbare, auf der rechten der aus dem Mobiltelefon herausragende Teil.

## Patentansprüche

1. Datenübertragungseinheit zur drahtlosen Kommunikation mit einem elektromedizinischen Implantat und einer Datenerfassungs- und -auswertezentrale (Home Monitoring Service Center)
**dadurch gekennzeichnet,**
**dass** die Datenübertragungseinheit eine in ein Basisgerät, insbesondere in ein Mobiltelefon (200), einführbare elektronische Steckkarte (100) ist, die wenigstens
eine drahtlose Datenschnittstelle (140) zum Austausch von Informationen mit einem elektromedizinischen Implantat,
eine drahtgebundene Datenschnittstelle (110) zum Basisgerät für den Austausch von Informationen zwischen Steckkarte (100) und Basisgerät,
mindestens eine Kontrolleinheit (120) zur Steuerung des Datenaustausches über wenigstens die drahtlose Datenschnittstelle (110)
sowie einen durch das Basisgerät über die drahtgebundene Datenschnittstelle (110) lesbaren Schreib/Lesedatenspeicher (130) aufweist,
wobei
die Kontrolleinheit (120) ausgebildet ist, über die drahtlose Datenschnittstelle (110) von einem externen Gerät empfangene Daten in den Schreib/Lesedatenspeicher (130) zu schreiben.

2. Datenübertragungseinheit nach Anspruch 1, **dadurch gekennzeichnet,**
**dass** der durch das Basisgerät über die drahtgebundene Datenschnittstelle (110) lesbare Schreib/Lesedatenspeicher (130) als nichtflüchtiger Schreib/Lesedatenspeicher ausgeführt ist.

3. Datenübertragungseinheit nach einem der Ansprüche 1 oder 2, **dadurch gekennzeichnet,**
**dass** die elektronische Steckkarte (100) die Vorgaben des MMC-Standards (MultiMedia Card), des SD-Standards (Secure Digital), des SM-Standards (SmartMedia), des MemoryStick-Standards oder des CF-Standards (CompactFlash) erfüllt.

4. Datenübertragungseinheit nach einem der vorhergehenden Ansprüche,
**dadurch gekennzeichnet,**
**dass** die Datenübertragungseinheit über eine Kodiereinheit verfügt, die mit der drahtgebundenen (110) und drahtlosen (140) Datenschnittstelle direkt oder mittelbar verbunden und ausgebildet ist, über die drahtlose Datenschnittstelle (140) von einem elektromedizinischen Implantat erfasste medizinische Daten oder technische Daten das elektromedizinische Implantat selbst betreffend derart zu verschlüsseln, dass die verschlüsselten Daten über die drahtgebundene Datenschnittstelle (110) auslesbar sind.

5. Datenübertragungseinheit nach einem der vorhergehenden Ansprüche,
**dadurch gekennzeichnet,**
**dass** die Datenübertragungseinheit über eine Dekodiereinheit verfügt, die mit der drahtgebundenen Datenschnittstelle (110) direkt oder mittelbar verbunden und ausgebildet ist, über die drahtgebundene Datenschnittstelle (110) empfangene Daten zu entschlüsseln.

6. Datenübertragungseinheit nach Anspruch 5, **dadurch gekennzeichnet, dass** die Dekodiereinheit direkt oder mittelbar mit der drahtlosen Datenschnittstelle (140) verbunden und ausgebildet ist, über die drahtgebundene Datenschnittstelle (110) empfangene kodierte oder verschlüsselte Parameter für den Betrieb eines elektromedizinischen Implantats vor deren Übertragung über die drahtlose Datenschnittstelle (140) an ein solches Implantat auf die Integrität der Parameter und ihre korrekte Übertragung zur Datenübertragungseinheit zu überprüfen.

7. Datenübertragungseinheit nach Anspruch 5, **dadurch gekennzeichnet,**
**dass** die Dekodiereinheit ausgebildet ist, über die drahtgebundene Datenschnittstelle (110) empfangene kodierte oder verschlüsselte Betriebssoftware für den Betrieb der Datenübertragungseinheit in einem Basisgerät auf die Integrität der Betriebssoftware und ihre korrekte Übertragung zur Datenübertragungseinheit zu überprüfen.

8. Datenübertragungseinheit nach einem der vorhergehenden Ansprüche,
**dadurch gekennzeichnet,**
**dass** die drahtlose Schnittstelle (140) ausgebildet ist, Daten bei einer Trägerfrequenz von 401 bis 406 MHz zu übertragen.

9. Datenübertragungseinheit nach einem der vorhergehenden Ansprüche,
**dadurch gekennzeichnet,**
**dass** der Schreib/Lesedatenspeicher (130) über die drahtgebundene Datenschnittstelle (110) nicht beschreibbar ist.

10. Datenübertragungseinheit nach einem der Ansprüche 1 bis 8, **dadurch gekennzeichnet, dass** der Schreib/Lesedatenspeicher (130) über die drahtgebundene Datenschnittstelle (110) beschreibbar ist.

11. Datenübertragungseinheit nach einem der vorhergehenden Ansprüche,
**dadurch gekennzeichnet,**
**dass** der Schreib/Lesedatenspeicher (130) als dual-ported RAM ausgeführt ist.

12. Datenübertragungseinheit nach einem der vorhergehenden Ansprüche,
**dadurch gekennzeichnet,**
**dass** der Schreib/Lesedatenspeicher (130) zwischen die drahtlose (140) und die drahtgebundene Datenschnittstelle (110) geschaltet ist.

13. Datenübertragungseinheit nach einem der vorhergehenden Ansprüche,
**dadurch gekennzeichnet,**
**dass** die elektronische Steckkarte (100) einen geeigneten Energiespeicher oder -puffer enthält, der die elektronischen Komponenten der Steckkarte (100) kurzzeitig mit Strom versorgt, wenn die Stromversorgung der Steckkarte (100) durch das Basisgerät unterbrochen wird.

14. Datenübertragungseinheit nach einem der vorhergehenden Ansprüche,
**dadurch gekennzeichnet,**
**dass** die elektronische Steckkarte (100) eine optische Anzeige (180) aufweist, die bei in ein Mobiltelefon (200) eingesteckter elektronischer Steckkarte (100) sichtbar ist und ausgebildet ist, ein Signal abzugeben, wenn ein Datenaustausch über die drahtlose Datenschnittstelle (140) stattfindet.

15. Datenübertragungseinheit nach einem der vorhergehenden Ansprüche,
**dadurch gekennzeichnet,**
**dass** die elektronische Steckkarte (100) einen akustischen Signalgeber aufweist, der ausgebildet ist, ein hörbares Signal abzugeben, wenn ein Datenaustausch über die drahtlose Datenschnittstelle (140) stattfindet.

16. Datenübertragungseinheit nach einem der vorhergehenden Ansprüche,
**dadurch gekennzeichnet,**
**dass** die drahtgebundene Datenschnittstelle (110) so ausgebildet ist, dass sich die elektronische Steckkarte (100) wie eine standardgemäße Datenspeicherkarte steuern und benutzen lässt.

17. Datenübertragungseinheit nach einem der vorhergehenden Ansprüche,
**dadurch gekennzeichnet,**
**dass** die Kontrolleinheit (120) der elektronischen Steckkarte (100) ausgebildet ist, ein gleichzeitiges Auslesen und Beschreiben des Schreib/Lesedatenspeichers (130) zu verhindern oder gleichzeitige Schreib- und Lesezugriffe so zu koordinieren, dass die ausgelesenen Daten in ihrer Gesamtheit entweder identisch mit jenen Daten sind, die vor Beginn des Schreibzugriffes im Schreib/Lesedatenspeicher (130) vorlagen, oder identisch mit jenen, die nach Ende des Schreibzugriffes im Schreib/Lesedatenspeicher (130) vorliegen.

18. Datenübertragungseinheit nach einem der vorhergehenden Ansprüche,
**dadurch gekennzeichnet, dass** die elektronische Steckkarte (100) zusätzlich einen zweiten Schreib/Lesedatenspeicher umfasst, der ausgebildet ist, eine Kopie der vor einem in Durchführung befindlichen Schreibzugriff auf den ersten Schreib/Lesedatenspeicher (130) in diesem enthaltenen Information für gleichzeitige Lesezugriffe über die jeweils andere Datenschnittstelle bereitzustellen.

19. Datenübertragungseinheit nach einem der vorhergehenden Ansprüche,
**dadurch gekennzeichnet,**
**dass** die elektronische Steckkarte (100) über ein bei eingesteckter Steckkarte (100) bedienbares Bedienelement (190) verfügt, durch das eine Datenübertragung über eine der Datenschnittstellen (110, 140) ausgelöst werden kann.

20. Datenübertragungseinheit nach einem der vorhergehenden Ansprüche,
**dadurch gekennzeichnet,**
**dass** die elektronische Steckkarte (100) ein ROM (150) oder Flash-ROM mit Programminstruktionen und -daten (300) zur Ausführung durch die Kontrolleinheit (120) enthält.

21. Datenübertragungseinheit nach einem der vorhergehenden Ansprüche,
**dadurch gekennzeichnet,**
**dass** die elektronische Steckkarte (100) ein ROM (150) oder Flash-ROM mit Programminstruktionen und -daten (310) zur Ausführung durch das Basisgerät enthält.

22. Datenübertragungseinheit nach einem der vorhergehenden Ansprüche,
**dadurch gekennzeichnet,**
**dass** die elektronische Steckkarte (100) ein RAM (160) zum Zwischenspeichern von Daten, die bei der Ausführung von Programminstruktionen (300) anfallen, aufweist.
